# EUROPEAN PATENT SPECIFICATION

(11) **EP 1 971 592 B1**
(45) Date of publication and mention of the grant of the patent: **27.04.2011**
(21) Application number: 06850470.3
(22) Date of filing: 12.12.2006
(51) Int. Cl.: C07D 333/20

(54) **IMPROVED SYNTHESIS AND PREPARATIONS OF DULOXETINE SALTS**
VERBESSERTE SYNTHESE UND ZUBEREITUNGEN VON DULOXETINSALZEN
PROCÉDÉ PERFECTIONNÉ DE SYNTHÈSE ET DE PRÉEPARATIONS DE SELS DE DULOXÉTINE

(30) Priority: 12.12.2005 US 749095 P; 12.12.2005 US 749096 P; 12.12.2005 US 749097 P; 23.06.2006 US 815835 P; 23.06.2006 US 815854 P; 23.06.2006 US 815856 P
(43) Date of publication of application: 24.09.2008
(73) Proprietor: Medichem, S.A., 08970 Sant Joan Despi, Barcelona (ES)
(72) Inventor: WINTER, Stephen, Barcelona (ES)
(74) Representative: Weiss, Wolfgang
(86) International application number: PCT/IB2006/004250
(87) International publication number: WO 2007/119114

(56) References cited:
- WO-A-2004/056795
- WO-A-2005/108386
- WO-A-2006/099433

## Description

### BACKGROUND OF THE INVENTION

### Field of the Invention

The invention relates to an improved process for the preparation of duloxetine hydrochloride. More particularly, the invention relates to a process for the enantiomeric enrichment of racemic duloxetine, and to a process for increasing the enantiomeric excess of enantiomerically enriched duloxetine.

### Discussion of the Related Art

Duloxetine hydrochloride (Compound 1) is the international commonly accepted name for N-methyl-N-[(3S)-(3-(l-naphthyloxy)-3-thien-2-yl)propyl]amine hydrochloride (which is also known as methyl-[(S)3-(naphthalen-1-yloxy)-3-thiophen-2-yl-propyl]-amine hydrochloride) and has an empirical formula of C₁₈H₁₉NOS·HCl and a molecular weight of 333.88. Duloxetine hydrochloride is a commercially marketed pharmaceutically active substance known to be useful for the treatment of major depressive disorder.

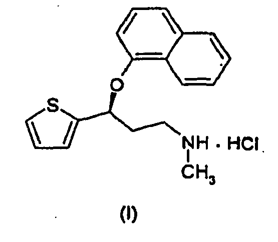

Duloxetine hydrochloride is a selective serotonin and norepinephrine reuptake inhibitor (SSNRI) for oral administration. In the United States, duloxetine hydrochloride is marketed under the name Cymbalta^{®} for the treatment of major depressive disorder and diabetic peripheral neuropathic pain. In Europe, duloxetine hydrochloride has been approved for the treatment of major depressive disorder and also for the treatment of moderate to severe stress urinary incontinence.

Duloxetine and its pharmaceutically acceptable salts are disclosed in U.S. Patent No. 5,023,269 ("the '269 patent"). No examples related to the preparation of (S)-duloxetine, or one of its pharmaceutically acceptable salts (*e.g*., the hydrochloride salt), are disclosed. In the '269 patent, racemic duloxetine was prepared by demethylating the corresponding N,N-dimethylpropanamine derivative using phenyl chloroformate to yield the corresponding carbamate as an intermediate. The carbamate was then hydrolyzed to afford racemic duloxetine as an oil, and was subsequently isolated as the oxalate salt. The '269 patent indicates that optically active isomers may be prepared from their corresponding optically active precursors by the described procedures, or by resolving the racemic mixtures. The process disclosed in the '269 patent for obtaining racemic duloxetine is shown in Scheme 1.

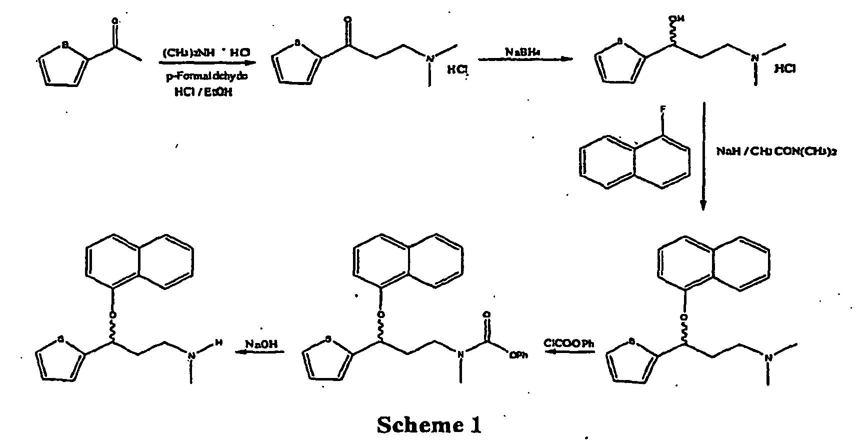

U.S. Patent No. 5,362,886 ("the '886 patent") discloses the above synthetic strategy but using the chiral intermediate of (S)-3-dimethylamino-1-(2-thienyl)-1-propanol. By using this chiral intermediate, (S)-duloxetine is obtained. The '886 patent provides the preparation of (S)-duloxetine in the form of its hydrochloride salt.

Methods for producing duloxetine and/or its salts are also disclosed in various other references, including: WO 03/070720; WO 04/065376; WO 04/011452; WO 04/024708; WO 04/005307; JP 2004123596; WO 04/13123; WO 04/005220; and WO 00/61540. Preparation of duloxetine hydrochloride is specifically described in U.S. Patent No. 5,491,243; WO 04/056795; J. Labelled Compd. Radiopharm., 36, 213 (1995); and Zhongguo Xinyao Zazhi, 14(1), 74-76 (2005). Generally, these alternative processes include resolution of a key intermediate or a stereoselective synthesis usually involving a stereospecific reduction of a keto group to give the corresponding alcohol.

Patent application WO 04/056795 discloses the preparation of(S)-duloxetine by resolution of racemic duloxetine using a chiral acid, and duloxetine hydrochloride is prepared by trituration from acetone.

Notably, in none of the examples described in the literature and/or outlined above are the crystal properties of duloxetine hydrochloride characterized or is the enantiomeric excess reported.

Here, it has been observed for the first time that duloxetine hydrochloride exists as a conglomerate. Solid duloxetine hydrochloride therefore prefers to exist in distinct enantiomerically-defined forms. This occurrence is rare and only expected in approximately between 5 and 10% of compounds. *See, e.g.,* J. Jacques, A. Collect and S.H. Wilden, ENANTIOMERS, RACEMATES AND RESOLUTIONS, Krieger Publishing Company (1991) and K. Saigo et. al., J. Am. Chem. Soc., 118, 3441-3449 (1996). Consequently, the solid has excellent crystallization characteristics (*i.e*., the compound may be enriched in one enantiomer by crystallization leaving racemic liquors). The advantage of increasing the enantiomeric excess is evident given (a) the relatively low cost of the achiral acid (as opposed to a chiral acid) and (b) that conglomerate resolution may be operated as a continuous process which is often extremely efficient for multi-ton manufacture. *See, e.g.,* J. Crosby, Tetrahedron Report Number 293, Tetrahedron, 47, 4789 (1991). An example of such a process is described in U.S. Patent No. 6,087,495 in which galanthamine hydrobromide is identified as a conglomerate and resolved by direct crystallization techniques. For processes relating to the separation of conglomerates, *see* Crosby, CHIRALITY AND INDUSTRY, Wiley, Chichester, 24-27 (1992).

Identification of a conglomerate is achieved by analysis of the physical characteristics of the solid form. Examples of such techniques include, but are not limited to, infra-red spectroscopy, powder x-ray diffraction and differential scanning calorimetry. In such techniques, a racemic.sample is usually compared to a sample that has been enantiomerically enriched. In the foregoing techniques, conglomerate samples will be identical in all enantiomeric compositions. *See; e.g.,* Jacques, ENANTIOMERS, RACEMATES AND RESOLUTIONS, at 53.

In addition to determining that duloxetine hydrochloride exists as a conglomerate, it has been observed that racemic duloxetine hydrochloride has a lower melting point than the single enantiomer form.

### SUMMARY OF THE INVENTION

The invention relates to an improved process for the preparation of duloxetine hydrochloride. More particularly, the invention relates to a process for the enantiomeric enrichment of racemic duloxetine, and to a process for increasing the enantiomeric excess of enantiomerically enriched duloxetine.

One aspect of the invention includes a process for the separation of the enantiomers of duloxetine HCl by direct crystallization. In such a process, it has been observed that it is not necessary to resolve racemic duloxetine using a chiral acid to prepare (S)-duloxetine from (±)-duloxetine.

Another aspect of the invention includes a method for the enantiomeric enrichment of racemic duloxetine by means of salt formation with an achiral acid, in which the method includes seeding a supersaturated solution of said salt with a sample of an enantiomerically enriched salt, and recovering the precipitated solid.

Another aspect of the invention includes a process for increasing the enantiomeric excess of a partially-resolved sample of duloxetine or salt thereof in which the acidic counterion is an achiral acid, and recovery of the precipitated solid. This process may optionally further include seeding a solution of the duloxetine or salt thereof with an enantiomerically-enriched duloxetine salt of the same acid.

Another aspect of the invention includes the use of an enantiomerically-enriched duloxetine salt prepared by any of the above-described processes in a pharmaceutical formulation, including, preferably, the hydrochloride salt.

Another aspect of the invention includes the discovery that duloxetine hydrochloride exists as a conglomerate. As such, the invention includes a process in which a supersaturated solution of racemic duloxetine hydrochloride is seeded with an enantiomerically-enriched sample to cause a favorable crystallization of duloxetine hydrochloride.

Herein also described is duloxetine hydrochloride acetone solvate, which is a new product formed when acetone is used during the process for the enantiomeric enrichment of duloxetine hydrochloride.

Herein also described is converting the duloxetine hydrochloride acetone solvate into duloxetine hydrochloride Form I by means of recrystallization.

### BRIEF DESCRIPTION OF THE DRAWINGS

The accompanying drawings, which are included to provide a further understanding of the invention and are incorporated in and constitute a part of this specification, illustrate embodiments of the invention and together with the description serve to explain the principles of the invention. In the drawings:
Figure 1 illustrates the powder XRD Spectrum of (S)-Duloxetine hydrochloride, Form I;

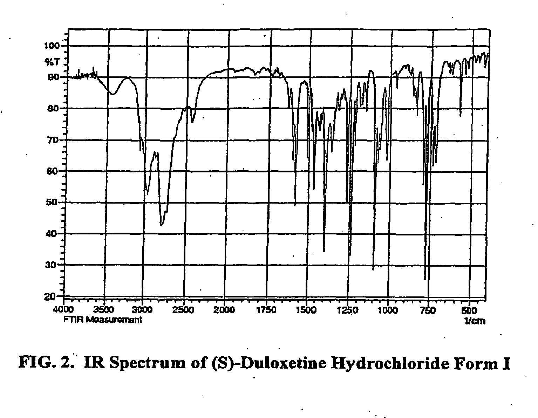

Figure 2 illustrates the IR Spectrum of (S)-Duloxetine hydrochloride, Form I;

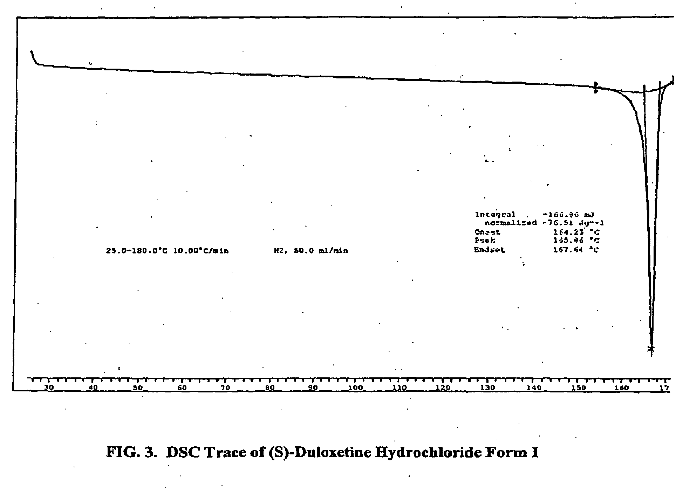

Figure 3 illustrates the DSC Trace of (S)-Duloxetine hydrochloride, Form I;
Figure 4 illustrates the powder XRD Spectrum of Duloxetine hydrochloride acetone solvate; and
Figure 5 illustrates the IR Spectrum ofDuloxetine hydrochloride acetone solvate.

### DETAILED DESCRIPTION OF THE PREFERRED EMBODIMENTS

Reference will now be made in detail to the preferred embodiments of the invention.

This invention may, however, be embodied in many different forms and should not be construed as limited to the embodiments set forth herein. In addition, and as will be appreciated by one of skill in the art, the invention may be embodied as a method, system or process.

The invention relates to an improved process for the preparation of duloxetine hydrochloride. More particularly, the invention relates to a process for the enantiomeric enrichment of racemic duloxetine, and to a process for increasing the enantiomeric excess of enantiomerically-enriched duloxetine.

One aspect of the invention includes a process for preparing a duloxetine salt in which the counterion is achiral, wherein the process includes the seeding of a supersaturated solution of racemic salt with an enantiomerically-enriched form of the salt, and recovery of the salt that crystallizes.

Another aspect of the invention includes a process for increasing the enantiomeric excess of an enantiomerically-enriched salt, wherein the process includes crystallization or solvent slurrying and recovery of the solid.

Another aspect of the invention includes a process for increasing the enantiomeric excess of a partially-resolved duloxetine salt, wherein the counterion is achiral and wherein the process includes crystallization or solvent slurrying and recovery of the solid, and in which the reduction in content of R-enantiomer is between approximately 0.5% and approximately 50%.

Herein also described is a crystallization method that provides duloxetine hydrochloride characterized by a powder X-ray spectrum having peaks at approximately 18.1, 18.9, 20.9, 23.4, 24.6, 28.0 ± 0.2 degrees 2θ and further peaks at approximately 9.7, 13.9, 14.5, 16.0, 22.7, 26.5, 27.4 ± 0.2 degrees 2θ.

In the above described processes, racemic or enantiomerically-enriched duloxetine may be prepared by any of the reported procedures or standard chemical methodologies. In those processes involving a seeding step, the enantiomeric excess of the seed should be high, and typically > 80% ee.

In the above described processes, the enantiomeric excess and yield of the product obtained will be dependent of the conditions used, such as solvent, concentration, temperature and pressure. Thus, in another aspect of the invention, the increase in enantiomeric excess observed may be approximately 50% ee or more and with approximately > 90% ee of final product after optimization. In another aspect of the invention, the enantiomeric excess of product will be approximately > 98% ee. In another aspect of the invention, the enantiomeric excess of product will be approximately > 99% ee. In another aspect of the invention, the enantiomeric excess of product will be approximately > 99.5% ee.

Another aspect of the invention includes a process that includes crystallizing duloxetine hydrochloride to obtain a product with a very high enantiomeric excess. In such process, it is preferable that the enantiomeric excess obtained by crystallization or successive crystallization(s) of duloxetine hydrochloride is approximately ≥ 99.0%, more preferably approximately ≥ 99.5%, and even more preferably is approximately ≥ 99.9%.

Another aspect of the invention includes formulations, and a process for preparing the same, of duloxetine hydrochloride having an (R)-duloxetine hydrochloride content lower than approximately 1% area by HPLC.

Another aspect of the invention includes formulations, and a process for preparing the same, of duloxetine hydrochloride having an (R)-duloxetine hydrochloride content lower than approximately 0.5% area by HPLC.

Another aspect of the invention includes formulations, and a process for preparing the same, of duloxetine hydrochloride having an (R)-duloxetine hydrochloride content lower than approximately 0.1% area by HPLC.

Herein also described is duloxetine hydrochloride as a solvate.

Herein also described is oxetine hydrochloride as a solvate with acetone.

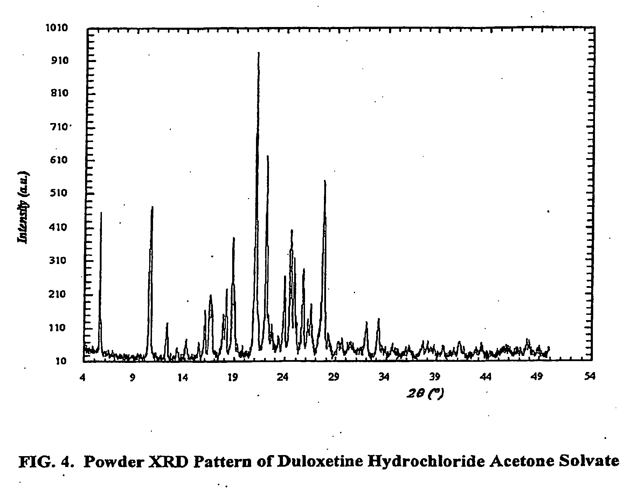

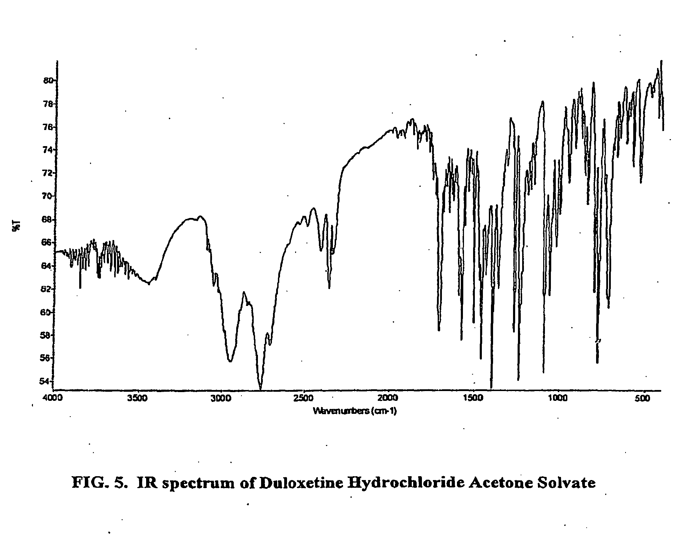

The duloxetine hydrochloride acetone solvate is characterized by X- Ray powder diffraction pattern (2θ) (± 0.2°) (XRD) having its main peaks at approximately 5.5, 10.5, 12.2, 16.6, 17.9, 18.2, 18.8, 21.1, 22.1, 23.9, 24.5, 24.8, 25.7, 27.7 ± 0.2 degrees 2θ, and further peaks at approximately 13.2, 14.1, 15.4, 16.0, 19.3, 22.6, 23.3, 26.2, 26.5, 28.2, 29.2, 29.6, 32.0, 33.2, 34.5, 36.2, 37.5, 38.0, 38.7, 39.6, 41.2, 42.8, 43.4, 47.7, 49.0° as illustrated in Figure 4. The duloxetine hydrochloride acetone solvate is further characterized by the IR spectrum illustrated in Figure 5.

Herein also described is converting the duloxetine hydrochloride acetone solvate into duloxetine hydrochloride Form I by means of recrystallization.

It will be apparent to those skilled in the art that various modifications and variations can be made in the present invention and specific examples provided herein without departing from the spirit or scope of the invention. Thus, it is intended that the present invention covers the modifications and variations of this invention that come within the scope of any claims and their equivalents.

### Specific Examples

The following examples are for illustrative purposes only and are not intended, nor should they be interpreted to, limit the scope of the invention.

### General Experimental Conditions:

### HPLC Method

### a. Chromatographic Purity HPLC Method

The chromatographic separation is carried out in a Phenomenex Luna C 18, 5 µm, 4.6 x 150 mm column at room temperature (20-25° C).

The mobile phase was prepared by mixing 500 mL of acetonitrile with 500 mL of buffer (pH = 2), which was prepared by dissolving 18.40 g of hexafluorophosphate in 1000 mL of water. The pH was adjusted to 2 with phosphoric acid. The mobile phase is mixed and filtered through 0.22 µm nylon membrane under vacuum.

The chromatograph was equipped with a 220 nm detector and the flow rate was 1 mL per minute. Test samples (10 µL) were prepared by dissolving the appropriate amount of sample in the mobile phase in order to obtain 0.5 mg per mL. The chromatogram was run for at least 30 minutes.

### b. HPLC Chiral Method

The chromatographic separation was carried out in a Daicel CHIRALCEL OD-RH, 5 µm, 4.6 x 150 mm column at room temperature (20-25° C).

The mobile phase was prepared by mixing 600 mL of acetonitrile with 400 mL of buffer (pH = 2) which was prepared from 18.40 g of hexafluorophosphate dissolved in 1000 mL of water. The pH was adjusted to 2 with phosphoric acid. The mobile phase was mixed and filtered through a 0.22 µm nylon membrane under vacuum.

The chromatograph was equipped with a 216 nm detector and the flow rate was 0.5 mL per minute. Test samples (5 µL) were prepared by dissolving the appropriate amount of sample in the mobile phase in order to obtain 0.5 mg of sample per mL. The chromatogram was run for at least 25 minutes.

### EXAMPLE 1: Preparation of (S)-N-methyl-(3-(1-naphthyloxy)-3-thien-2-yl)propylamine hydrochloride (Duloxetine hydrochloride) starting from chloroethyl carbamate

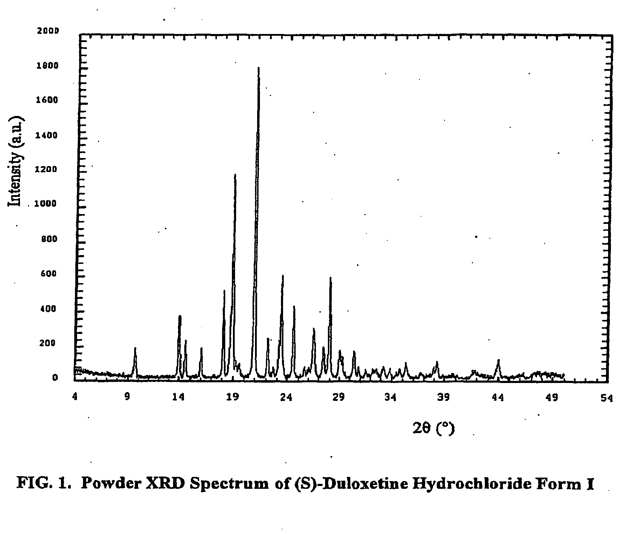

(*S*)-*N*-Methyl-[3-(naphthalen-1-yloxy)-3-thiophen-2-yl-propyl]-carbamic acid 1-chloroethyl ester (6.42 g, 60% ee) was stirred in methanol (3.2 mL) at ambient temperature for 16 hours to give a solution of duloxetine hydrochloride in methanol. Acetone (30 mL) was then added, and the mixture was seeded with 5 mg of duloxetine hydrochloride of 99.3% ee. The mixture was stirred at ambient temperature for 1 hour and then maintained at 6° C for 16 hours. The resulting solid was then filtered, washed with acetone (5 mL), and dried in a vacuum at ambient temperature for 24 hours to yield 1.56 g of duloxetine hydrochloride Form I. Analysis: Yield: 29%; 98% ee; XRD: substantially the same as Figure 1). The filtrate liquors were concentrated and dissolved in acetone (5 mL). After being maintained at 6° C for 16 hours, the liquors yielded an additional 0.486 g of duloxetine hydrochloride as a solid after filtration. Analysis: Yield: 9%, 94% ee. Analysis of the liquors gave < 5% ee.

### EXAMPLE 2: Preparation of (S)-N-methyl-(3-(1-naphthyloxy)-3-thien-2-yl)propylamine hydrochloride (Duloxetine hydrochloride) starting from (S)-N-methyl-(3-(1-naphthyloxy)-3-thien-2-yl)propylamine (Duloxetine Base)

Duloxetine base (1 g, 62% ee) was stirred in ethyl acetate (4 mL) at 0° C and hydrochloric acid in methanol (1.25 M, 2.42 mL) was added. The mixture was stirred at this temperature for 4 hours and then at ambient temperature for an additional 16 hours. The mixture was then evaporated, and acetone (5 mL) was added which caused abundant precipitation. Additional acetone (7 mL) was added, and the mixture was stirred for an additional 30 minutes and filtered. The product was then washed with acetone (2 × 5 ml) and dried under vacuum at 50° C to yield 0.43 g of duloxetine hydrochloride as a white solid. Analysis: 97.4% ee; >97 % peak area duloxetine hydrochloride, XRD: substantially the same as Figure 1.

### EXAMPLE 3: Duloxetine Hydrochloride Purification

Crude duloxetine HCl (23.38 g, 59% ee) was dissolved in acetone (110 mL), and stirred art 0° C for 20 hours. The suspension was then stirred at this temperature for an additional hour, filtered, and the resulting solid dried under vacuum at 40 ° C to yield 11.35 g of duloxetine HCl. Analysis: 70.1%ee; >98.9 % peak area duloxetine HCl; < 0.01% (peak area) 4-(3-methylamino-1-thiophen-2-yl-propyl)-naphthalen-1-ol.

### EXAMPLE 4: Duloxetine Hydrochloride Purification

Crude duloxetine HCl (1.3 g, 59% ee) was dissolved in acetone (10 mL), and cooled to 0° C causing precipitation. The mixture was warmed to ambient temperature and the suspension was stirred overnight. The mixture was filtered, and the solid dried under vacuum at 40° C to yield 0.34 g of duloxetine HCl. Analysis: 98.9% ee, >99.3 % peak area duloxetine HCl; < 0.01% (peak area) 4-(3-methylamino-1-thiophen-2-yl-propylynaphthalen-1-ol.

### EXAMPLE 5: Preparation of racemic N-methyl-(3-(1-naphthyloxy)-3-thien-2-yl)propylamine hydrochloride (racemic Duloxetine hydrochloride)

Duloxetine hydrochloride (5.0 g, 15 mmol) and sodium tert-pentoxide 40% in toluene (14 mL, 3 eq) were heated in DMSO (50 inL) for 45 h at 60° C. The mixture was cooled, quenched with water (40 mL), and extracted with iso-propylacetate (2 × 30mL). The organic layers were then combined, washed with water (20 mL), and evaporated to give 5.13 g of a racemic oil according to chiral HPLC analysis. The residue was then dissolved in acetone (50 mL), stirred at 0° C for 10 minutes, and hydrochloric acid in ether (2 M, 6 mL, 12 mmol, 0.8 eq) was added. The mixture was stirred at this temperature for 2 hours and then filtered. The product was then washed with acetone (2 × 5 ml) and dried under vacuum at 40° C to yield 2.0 g of duloxetine hydrochloride. The solid was identical according to IR, XRPD (Form I), achiral HPLC and ¹H NMR to samples of single enantiomer Duloxetine hydrochloride. According to chiral HPLC analysis, however, the solid was racemic. Analysis: Mp 133.9 -134.4° C (*cf*. single enantiomer 165.7-166.0° C).

### EXAMPLE 6: Direct Resolution of Racemic N-methyl-(3-(1-naphtha yloxy)-3-thien-2-yl)propylamine hydrochloride (Duloxetine Hydrochloride)

Racemic duloxetine hydrochloride (0.536 g) was dissolved with heating in acetone (5 mL) and allowed to cool. After 30 minutes, 1 mg of (S)-duloxetine hydrochloride was added (>99% ee) and the mixture was stirred for an additional hour. The resulting solid was collected by filtration, and dried under vacuum at 40° C to yield 0.16 g of duloxetine hydrochloride as a white solid. This solid exhibited 12.1% ee (S-enantiomer) by chiral HPLC. A sample of the filtrate was evaporated and analyzed and exhibited 8.2% ee (R-enantiomer).

### EXAMPLE 7 (Comparison): Conversion of Duloxetine Hydrochloride Acetone Solvate to Form I by Recrystallisation

Duloxetine hydrochloride acetone solvate (4.05 Kg) was recrystallised from ethanol (8.4 Kg) with a hot filtration. The mixture was cooled to between 0 and 5° C and stirred at this temperature for 2 hours. The mixture was then filtered and dried at 40° C for 13 hours hours under vacuum to yield 2.29 Kg of duloxetine hydrochloride, Form I. Analysis: powder XRD Form I.

### EXAMPLE 8 (Comparison): Conversion of Duloxetine Hydrochloride Acetone Solvate to Form I by Heating

Duloxetine hydrochloride acetone solvate (2.955 g) was heated under vacuum at 60° C for 16 hours to yield 2.515 g of duloxetine hydrochloride, Form I. Analysis: XRD and IR duloxetine hydrochloride Form I.

### EXAMPLE 9 (Comparison): Conversion of Duloxetine Hydrochloride Form I to Duloxetine Hydrochloride Acetone Solvate

Duloxetine hydrochloride, Form I (1 g) was stirred in 5 mL of acetone at 40° C for 1hour. The mixture was allowed to cool to ambient temperatures and was stirred for an additional 24 hours. Thereafter, the mixture was filtered and immediately subjected to analysis by powder XRD and IR. Yield: 1.04 g. Analysis: XRD and IR-duloxetine hydrochloride acetone solvate.

## Claims

1. A process for the enantiomeric enrichment of a duloxetine salt, in which the counterion of duloxetine is an achiral acid, comprising at least one of direct crystallization and solvent slurrying of a starting duloxetine salt to recover the enriched duloxetine salt remaining in a solid phase component, **characterized in that** the starting duloxetine salt contains less than about 99.5% of (S)-duloxetine.

2. The process of claim 1, wherein said duloxetine salt is duloxetine hydrochloride.

3. The process of claim 1, wherein said recovered duloxetine salt is (S)-duloxetine hydrochloride.

4. The process of claim 3, wherein said recovered duloxetine salt contains less than approximately 1% area by HPLC of(R)-duloxetine hydrochloride.

5. The process of claim 3, wherein said recovered duloxetine salt contains less than approximately 0.5% area by HPLC of (R)-duloxetine hydrochloride.

6. The process of claim 3, wherein said recovered duloxetine salt contains less than approximately 0.1% area by HPLC of (R)-duloxctinc hydrochloride.

7. A formulation containing said recovered salt prepared according to the process of any of claims 3 to 6.

8. The formulation of claim 7, further comprising at least one excipient material.

9. The process of claim 1, wherein the starting duloxetine is racemic.

10. The process of claim 1, wherein the starting duloxetine salt contains less than about 90% of (S)-duloxetine.

11. The process of claim 1, wherein the starting duloxetine salt contains less than about 85% of (S)-duloxetine.

12. The process of claim 1, further comprising the step of seeding a supersaturated solution of said starting duloxetine salt with an enantiomerically-enriched form of the salt.

## Patentansprüche

1. Verfahren für die enantiomere Anreicherung eines Duloxetinsalzes, wobei das Gegenion von Duloxetin eine achirale Säure ist, umfassend mindestens eines aus direkter Kristallisation und Lösungsmittel-Aufschlämmen eines Ausgangs-Duloxetinsalzes, um das angereicherte Duloxetinsalz, das in einem Festphasen-Komponenten verbleibt, zurückzugewinnen, **gekennzeichnet dadurch, dass** das Ausgangs-Duloxetinsalz weniger als etwa 99,5% (S)-Duloxetin enthält.

2. Verfahren nach Anspruch 1, wobei das Duloxetinsalz Duloxetin-Hydrochlorid ist.

3. Verfahren nach Anspruch 1, wobei das zurückgewonnene Duloxetinsalz (S)-Duloxetin-Hydrochlorid ist.

4. Verfahren nach Anspruch 3, wobei das zurückgewonnene Duloxetinsalz weniger als ungefähr 1 Flächen-% nach HPLC (R)-Duloxetin-Hydrochiorid enthält.

5. Verfahren nach Anspruch 3, wobei das zurückgewonnene Duloxetinsalz weniger als ungefähr 0,5 Flächen-% nach HPLC (R)-Duloxetin=Hydrochlorid enthält.

6. Verfahren nach Anspruch 3, wobei das zurückgewonnene Duloxetinsalz weniger als ungefähr 0,1 Flächen-% nach HPLC (R)-Duloxetin-Hydrochlorid enthält.

7. Formulierung enthaltend das zurückgewonnene Salz, das gemäß des Verfahrens nach einem der Ansprüche 3 bis 6 hergestellt wurde.

8. Formulierung nach Anspruch 7, weiter umfassend mindestens ein Hilfsstoffmaterial.

9. Verfahren nach Anspruch 1, wobei das Ausgangs-Duloxetin racemisch ist.

10. Verfahren nach Anspruch 1, wobei das Ausgangs-Duloxetinsalz weniger als etwa 90% (S)-Duloxetin enthält.

11. Verfahren nach Anspruch 1, wobei das Ausgangs-Duloxetinsalz weniger als etwa 85% (S)-Duloxetin enthält.

12. Verfahren nach Anspruch 1, weiter umfassend den Schritt Impfen einer übersättigten Lösung des Ausgangs-Dulotexinsalzes mit einer enantiomer-angereicherten Form des Salzes.

## Revendications

1. Procédé pour l'enrichissement énantiomérique d'un sel de duloxétine, dans lequel le contre-ion de la duloxétine est un acide achiral, comprenant au moins l'une de la cristallisation directe et de la mise en suspension dans un solvant d'un sel de duloxétine de départ pour récupérer le sel de duloxétine enrichi restant dans un composant en phase solide, **caractérisé en ce que** le sel de duloxétine de départ contient moins d'environ 99,5 % de (S)-duloxétine.

2. Procédé selon la revendication 1 où ledit sel de duloxétine est le chlorhydrate de duloxétine.

3. Procédé selon la revendication 1 où ledit sel de duloxétine récupéré est le chlorhydrate de (S)-duloxétine.

4. Procédé selon la revendication 3 où ledit sel de duloxétine récupéré contient moins d'approximativement 1 % en aire par CLHP de chlorhydrate de (R)-duloxétine.

5. Procédé selon la revendication 3 où ledit sel de duloxétine récupéré contient moins d'approximativement 0,5 % en aire par CLHP de chlorhydrate de (R)-duloxétine.

6. Procédé selon la revendication 3 où ledit sel de duloxétine récupéré contient moins d'approximativement 0,1 % en aire par CLHP de chlorhydrate de (R)-duloxétine.

7. Formulation contenant ledit sel récupéré préparé selon le procédé selon l'une quelconque des revendications 3 à 6.

8. Formulation selon la revendication 7 comprenant en outre au moins un produit excipient.

9. Procédé selon la revendication 1 où la duloxétine de départ est racémique.

10. Procédé selon la revendication 1 où le sel de duloxétine de départ contient moins d'environ 90 % de (S)-duloxétine.

11. Procédé selon la revendication 1 où le sel de duloxétine de départ contient moins d'environ 85 % de (S)-duloxétine.

12. Procédé selon la revendication 1 comprenant en outre l'étape d'ensemencement d'une solution sursaturée dudit sel de duloxétine de départ avec une forme énantiomériquement enrichie du sel.
